# FASCICULE DE BREVET EUROPEEN

(11) **EP 0 323 770 B1**
(45) Date de publication et mention de la délivrance du brevet: **07.12.1994**
(21) Numéro de dépôt: 88403098.2
(22) Date de dépôt: 07.12.1988
(51) Int. Cl.: G06F 15/62

(54) **Procédé et dispositif permettant de reconstituer la forme et la position d'objets dans l'espace**
Verfahren und Einrichtung zur Form- und Lagewiederherstellung von Objekten im Raum
Method and apparatus allowing to reconstitute shape and position of objects in space

(30) Priorité: 09.12.1987 FR 8717140
(43) Date de publication de la demande: 12.07.1989
(73) Titulaire: Schlumberger, Etienne, F-75013 Paris (FR); Schlumberger, Maurice, F-38700 Le Sappey en Chartreuse (FR)
(72) Inventeur: Schlumberger, Etienne, F-75013 Paris (FR); Schlumberger, Maurice, F-38700 Le Sappey en Chartreuse (FR)
(74) Mandataire: Keib, Gérard

(56) Documents cités:
- COMPUTER VISION, GRAPHICS AND IMAGE PROCESSING,no. 3, septembre 1984, pages 249-265; A. KUBA" The reconstruction of two-directionally connected binary patterns from their two orthogonal projections"
- FUTURE GENERATIONS COMPUTER SYSTEMS, no. 5, septembre 1985, pages 353-375; M. KUWAHARA et al.: "Medical image processing: An overview of and case study in the diagnosis of cardiac diseases"

## Description

La présente invention concerne un procédé permettant de reconstituer la forme et la position d'objets dans l'espace à partir d'au moins trois projections de ces objets obtenues par transmission d'ondes influencées par le passage dans ces objets à partir de sources de ces ondes.

On entend par "objet" un ensemble continu, massif ou non, de caractéristiques physiques quasi constantes et homogènes, comme par exemple, des vaisseaux sanguins ou des os en imagerie médicale ou des filons en géographie.

L'invention vise également le dispositif pour la mise en oeuvre du procédé précité.

Les méthodes habituelles de positionnement d'un objet dans l'espace reposent sur l'observation, à partir de positions diverses, des contours extérieurs de l'objet à définir et situer.

Les exemples les plus communs sont issus directement de la vision stéréoscopique humaine. Une approche semblable se trouve dans d'autres domaines tels que la géophysique ou la médecine quelles que soient les ondes utilisées allant des rayonnements électro-magnétiques (rayons X, radar, vision classique), aux ondes sonores (sonar, échographies). Tous ces procédés utilisent dans tous les cas les brusques différences d'images (en atténuation ou en réflexion) pour positionner les contours de l'objet.

Ces différentes méthodes obligent, afin de positionner un objet avec précision, à le ceinturer d'un nombre suffisant de points d'observations, afin de définir son contour. Dans le cas d'objets complexes, ou irréguliers, ce nombre peut devenir très élevé, et leur disposition doit parfois entourer complètement l'objet comme, par exemple, dans le cas d'un scanner.

Dans le cas d'observations d'objets "translucides", où les ondes pénètrent l'objet sans être totalement absorbées, comme dans le cas d'images par rayons X, une information supplémentaire apparaît sur l'épaisseur de l'objet traversé. L'atténuation est, en effet, fonction du coefficient d'atténuation de la matière traversée, ainsi que de l'épaisseur de cette matière.

Comme indiqué plus haut, le même cas se retrouve lors de la transmission d'ondes de pressions, par exemple sismiques, mais alors il est aussi possible de faire intervenir les aspects de temps de parcours qui, eux aussi, dépendent de la nature et de l'épaisseur des milieux traversés.

Dans tous les cas, il apparaît donc une relation entre le phénomène observé et la distance parcourue dans l'objet.

Si on soumet un objet à des ondes qui le traversent et si on analyse l'image reçue, on peut obtenir une carte des modifications de ces ondes, qui sont fonction, en particulier, de la distance parcourue par un rayon dans l'objet.

Une image bien connue d'une telle carte est une radiographie. Sur celle-ci, plus un point est clair, par exemple, plus la distance parcourue dans l'objet est grande.

Tout système d'éclairage dans lequel il n'existe, par source, qu'un rayon clairement localisé par son origine et son point d'impact de position connue sur un cliché, et passant par un point de l'espace, permettra d'établir une carte de la modulation du faisceau éclairant fonction des distances parcourues dans l'objet.

Différents types de projection viennent naturellement à l'esprit :
- des projections à rayons parallèles, lorsque la source est à l'infini et que les récepteurs couvrent un plan de réception,
- des projections coniques, lorsque la source est à distance finie,
- des projections plus complexes, lorsque les sources et les récepteurs se trouvent sur des droites non sécantes, ni parallèles, ou encore toute autre combinaison qui permette de "couvrir" une partie de l'espace.

L'exemple le plus habituel d'une projection conique est un cliché de radiographie.

Un seul cliché radiographique ne permet pas de restituer la position spatiale d'un objet donné, bien que ce cliché offre beaucoup plus d'information à son observateur qu'un cliché "photographique", en particulier sur la structure interne de l'objet en question.

Si l'on veut obtenir plus d'informations sur l'intérieur de l'objet, il devient alors nécessaire d'en faire plusieurs clichés radiographiques, ainsi que le savent depuis longtemps tous les radiologues. Parfois, pour des objets même relativement simples, deux clichés ne suffisent pas, et il convient d'en prendre un nombre supérieur, pris de points précis.

Ainsi, dans le cas du scanner, on explore l'ensemble de la circonférence d'un objet, ce qui revient à effectuer une multitude de projections de l'objet afin de reconstruire sa forme et sa position dans l'espace.

Ce scanner est toutefois un appareil extrêmement complexe et coûteux.

Le but de la présente invention est de proposer un procédé qui permet de reconstruire la forme et la position d'objets dans l'espace à partir d'un nombre très réduit de projections (mais supérieur à deux projections) et qui puisse ainsi être simple et moins onéreux que dans le cas des procédés connus utilisant une multitude de projections.

Suivant l'invention, le procédé permettant de reconstituer la forme et la position d'objets dans l'espace à partir d'au moins trois projections de ces objets obtenues par transmission d'ondes influencées par le passage dans ces objets à partir de sources de ces ondes, le procédé étant divulgué dans la revendication 1.

La distance parcourue à travers un objet peut être déterminée sur chaque projection en mesurant par exemple la modification de l'onde lors du passage de celle-ci dans l'objet.

Le volume qui contient l'objet et les rayons qui traversent cet objet peuvent être déterminés avec précision. Ce volume permet donc de déterminer la forme et la position approximatives de l'objet.

Le procédé conforme à l'invention permet à partir de la connaissance des rayons et des distances parcourues par ces rayons dans l'objet, de déterminer un espace qui est nécessairement à l'extérieur de l'objet et un espace qui est nécessairement à l'intérieur de cet objet.

Les demandeurs ont établi qu'en répétant plusieurs fois les étapes E et F, l'écart entre l'espace extérieur à l'objet et l'espace intérieur à l'objet devient de plus en plus faible et donc ces espaces se confondent de plus en plus avec le contour réel de l'objet.

Ainsi, à condition de mesurer avec précision les distances et de déterminer avec précision la position d'un grand nombre de rayons, il est possible de reconstruire à partir d'un nombre réduit de projections de l'objet, la totalité de la forme de l'objet.

Le procédé conforme à l'invention utilise au moins trois projections de l'objet. L'écart entre l'espace nécessairement à l'extérieur de l'objet et celui nécessairement à l'intérieur de l'objet converge rapidement. Néanmoins, dans des cas complexes, un nombre de projections supérieur à trois pourrait être utile, ainsi qu'un choix judicieux de la position des sources et des récepteurs.

Selon l'invention, on détermine à partir de points connus du contour de l'objet, en particulier ceux correspondant aux points de tangence à l'objet de deux rayons issus de deux sources différentes, d'autres points du contour de ce dernier, en reportant à partir des points connus la distance parcourue dans cet objet par les rayons passant par ces points connus.

La connaissance de ces points permet de mieux définir la forme et la position de l'objet. Ainsi, en utilisant ces points connus, l'écart entre les deux espaces définis ci-dessus converge plus rapidement.

Le procédé selon l'invention peut s'appliquer facilement à la reconstruction de la forme et de la position de plusieurs objets adjacents.

A cet effet, on détermine le volume qui contient chacun des objets et les rayons issus des sources qui traversent cet objet, et on détermine à partir de ce volume la forme et la position de cet objet comme indiqué précédemment.

Le volume précité de chaque objet est déterminé par la discontinuité dans la transmission des rayons lorsque ceux-ci atteignent le contour apparent de l'objet.

Le procédé selon l'invention implique d'effectuer un grand nombre d'opérations mettant en oeuvre un grand nombre de distances et de rayons déterminés avec précision. Ce grand nombre d'opérations peut toutefois être réalisé au moyen d'un ordinateur de capacité moyenne.

Des tentatives ont été effectuées pour reconstituer un objet à partir de deux projections (voir COMPUTER VISION, GRAPHICS AND IMAGE PROCESSING n° 3, septembre 1984 A. KUBA). Ce procédé utilise en principe les étapes (A) à (H) divulgués dans la revendication 1.

Selon un autre aspect de l'invention, le dispositif pour la mise en oeuvre du procédé précité, comprend des moyens pour obtenir au moins trois projections de ces objets par transmission d'ondes influencées par le passage dans ces objets à partir de sources de ces ondes et des moyens comme divulgué dans la revendication 5.

Les moyens B à H peuvent être mis en oeuvre dans un ordinateur. Ainsi, le dispositif selon l'invention tel que revendiqué se compose essentiellement d'un système permettant d'obtenir au moins trois projections, de moyens pour mesurer les distances traversées dans l'objet par les différents rayons et d'un ordinateur.

Un tel dispositif est donc beaucoup plus simple et devrait nécessiter moins de rayonnement qu'un appareil tel qu'un scanner réalisant et analysant une multitude de projections.

D'autres particularités et avantages de l'invention apparaîtront encore dans la description ci-après.

Aux dessins annexés donnés à titre d'exemples non limitatifs :
- la figure 1 est une vue schématique montrant un objet et trois projections de celui-ci obtenues à partir de trois sources d'ondes traversant cet objet,
- la figure 2 est une vue schématique montrant un objet, deux projections de celui-ci perpendiculaires l'une à l'autre, obtenues à partir de deux sources d'ondes supposées à l'infini, et la coupe de cet objet par un plan passant par les sources,
- la figure 3 est une section d'un objet par un plan contenant deux séries de rayons perpendiculaires, encadrée par les rayons limites, dans ce plan, pour lesquels la distance parcourue dans l'objet est nulle,
- la figure 4 est une vue de l'espace intérieur de l'objet obtenu après report des distances parcourues dans cet objet par la première série de rayons, à partir des limites du volume contenant indiquées en figure 3,
- la figure 5 est une vue de l'espace intérieur de l'objet obtenu après report des distances parcourues dans cet objet par la seconde série de rayons,
- la figure 6 est une vue de l'espace obtenu par la combinaison des reports de distance effectués selon les figures 4 et 5,
- la figure 7 est une vue de l'espace extérieur à l'objet obtenu par report des distances parcourues dans cet objet à partir de l'espace intérieur obtenu selon la figure 6,
- la figure 8 est une vue du nouvel espace intérieur à l'objet obtenu à partir de l'espace extérieur selon la figure 7,
- la figure 9 est une vue du nouvel espace extérieur à l'objet obtenu à partir de l'espace intérieur selon la figure 8,
- la figure 10 est une vue montrant la méthode de détermination de nouveaux points à partir d'un point connu par report des distances parcourues dans l'objet selon deux directions perpendiculaires,
- la figure 11 est un schéma illustrant le procédé de reconstruction d'un objet contenu à l'intérieur d'un autre objet,
- la figure 12 est un schéma illustrant le cas de l'utilisation du contour apparent dans le cas d'une troisième projection,
- la figure 13 est un schéma illustrant le dispositif selon l'invention,
- la figure 14 est un diagramme illustrant les principales étapes du logiciel de simulation du procédé selon l'invention,
- les figures 15a, 15b et 15c sont des vues d'images-écrans obtenues avec le logiciel de simulation de la figure 14.

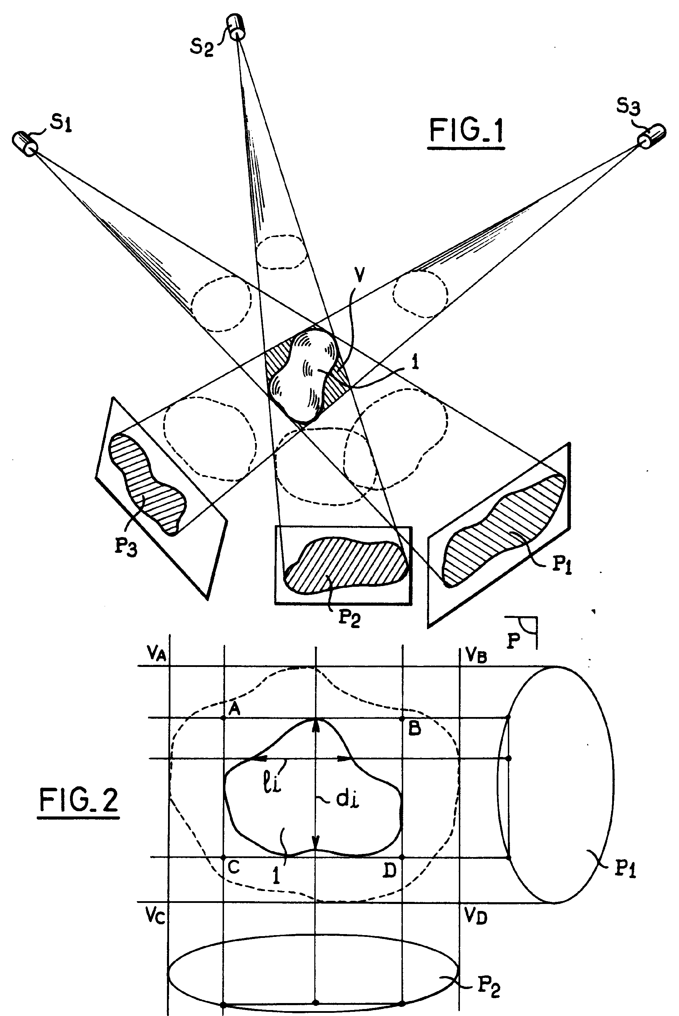

Sur la figure 1 la référence 1 désigne un objet dont un désire reconstituer la forme et la position dans l'espace selon le procédé conforme à l'invention.

P₁, P₂, P₃ désignent les projections de l'objet 1 obtenues par transmission d'ondes influencées par le passage dans cet objet à partir de trois sources S₁, S₂, S₃.

Ces ondes peuvent par exemple être des rayons X ou des ondes sonores. Les ondes subissent après leur passage dans l'objet 1 une modification qui est fonction de la distance parcourue dans cet objet.

On peut donc déterminer au moyen de capteurs appropriés, pour chaque point des projections P₁, P₂, P₃ la distance parcourue à travers l'objet 1 par chaque rayon issu d'une des sources S₁, S₂, S₃.

On voit sur la figure 1 que l'objet 1 est contenu dans un volume V qui contient tous les rayons issus des sources S₁, S₂, S₃ qui traversent cet objet 1.

La détermination de ce volume V fournit une première indication de la forme et de la position de l'objet 1.

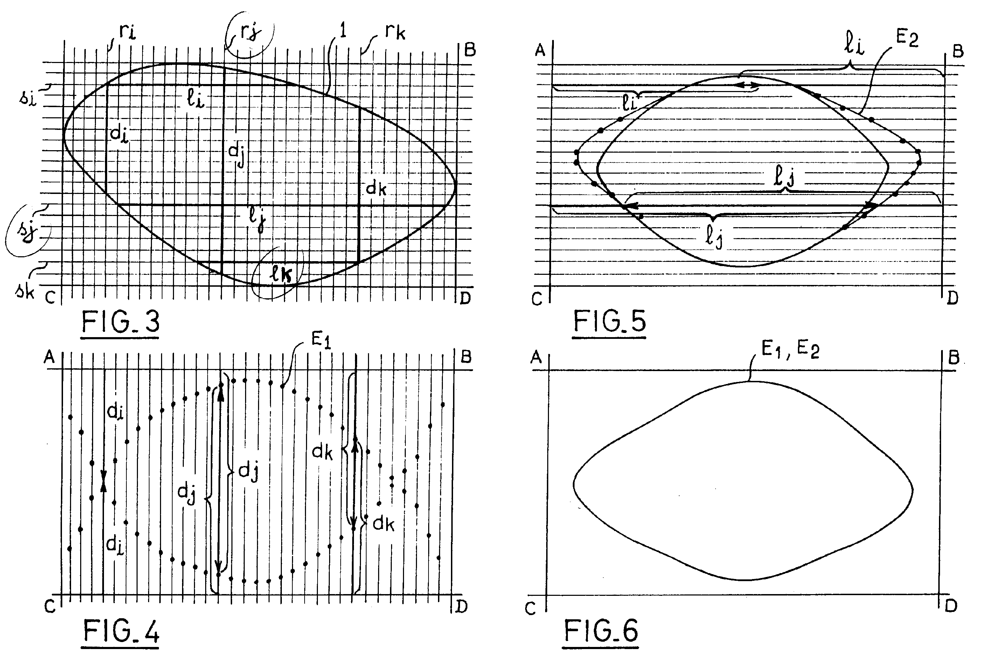

Pour simplifier l'exposé du procédé selon l'invention, on supposera que l'objet 1 est seulement traversé par deux séries perpendiculaires de rayons issus de sources situées à l'infini ou de deux séries de sources ponctuelles émettant chacune une série de rayons parallèles. Cette représentation est figurée sur la figure 2. L'objet 1 est contenu dans un volume qui contient tous les rayons issus des sources, qui traversent cet objet, schématisé par V_{A} V_{B} V_{C} V_{D}. L'intersection de ce volume avec le plan P de la figure 2 est un rectangle ABCD.

Pour simplifier l'exposé du procédé selon l'invention, on va décrire ci-après la méthode qui permet de reconstituer la forme et la position de l'objet 1 dans le plan P de la figure 2 à l'intérieur du rectangle ABCD. On a désigné par Lᵢ la distance parcourue dans l'objet 1 par un rayon parallèle à AB ou CD et par dᵢ la distance parcourue par un rayon parallèle à AC ou BD.

L'invention ci-après est illustrée, pour simplifier, uniquement par deux projections. Néanmoins trois projections sont nécessaires selon l'invention.

Comme déjà indiqué précédemment, le rectangle ABCD permet d'encadrer la forme et la position de l'objet 1 dans le plan P. Par ailleurs, il est possible de déterminer en analysant chaque point des projections P₁, P₂ les distances lᵢ et dᵢ parcourues dans l'objet par les rayons issus des sources.

Sur la figure 3 on a représenté la coupe d'un objet 1 contenu à l'intérieur d'un rectangle ABCD. Cet objet 1 est traversé par des rayons rᵢ, rⱼ, rₖ dans la direction parallèle à AC ou BD et par des rayons sᵢ, sⱼ, sₖ dans la direction parallèle à AB ou CD.

Les distances traversées par ces rayons sont respectivement dᵢ, dⱼ, dₖ pour rᵢ, rⱼ et rₖet lᵢ, lⱼ, lₖ pour sᵢ, sⱼ et sₖ.

On reporte les distances parcourues par les rayons dans l'objet 1 à partir des limites extérieures telles que AB, CD et AC, BD du quadrilatère ABCD qui contient l'objet 1 dans le cas de deux sources.

Le résultat de ces reports de distances est indiqué sur les figures 4 et 5. On obtient pour les reports de distances dᵢ, dⱼ, dₖ un espace E₁ (voir figure 4) et pour les distances lᵢ, lⱼ, lₖ un espace E₂ (voir figure 5). Ces espaces E₁ et E₂ correspondent au recouvrement de toutes ces distances.

En combinant ces deux espaces, on obtient un espace E₁, E₂ (voir figure 6) qui est nécessairement à l'intérieur de l'objet.

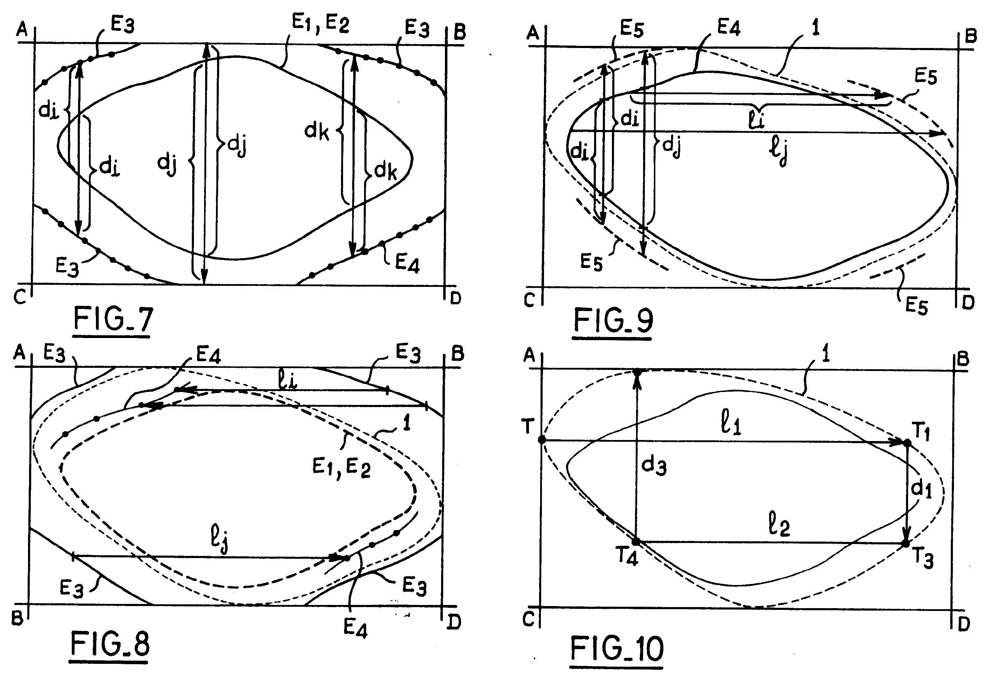

A ce stade du procédé, on sait donc que la limite de l'objet 1 à définir est située à l'intérieur du quadrilatère ABCD et à l'extérieur de l'espace E₁, E₂. Dans une étape ultérieure du procédé, on reporte les distances parcourues par les rayons dans l'objet à partir des limites extérieures de l'espace nécessairement intérieur E₁, E₂ (voir figure 7) pour définir un second espace E₃ qui est nécessairement à l'extérieur de l'objet 1 et qui est plus restreint que le quadrilatère ABCD.

On est donc parvenu à préciser davantage la forme et la position de l'objet 1.

Dans une étape ultérieure du procédé, on reporte les distances dᵢ, dⱼ ; lᵢ, lⱼ parcourues dans l'objet à partir des limites extérieures de l'espace E₃ précité (voir figure 8) pour définir un troisième espace E₄ qui est nécessairement à l'intérieur de l'objet 1 et qui est plus étendu que le premier espace E₁, E₂. La figure 8 montre donc que l'on est encore parvenu à préciser davantage la forme et la position de l'objet.

A partir de ce nouvel espace E₄ intérieur à l'objet, on détermine comme précédemment par report des distances dᵢ, dⱼ ; lᵢ, lⱼ (voir figure 9), un nouvel espace E₅ qui est nécessairement à l'extérieur de l'objet 1 (représenté en pointillé sur les figures 8 et 9) et qui est plus restreint que l'espace E₃ extérieur à l'objet précédemment défini.

On voit sur la figure 9 que l'écart entre les espaces intérieur E₄ et extérieur E₅ a encore diminué.

On reprend ensuite les étapes précédentes de façon à déterminer par récurrence des espaces extérieurs et intérieurs à l'objet 1 de plus en plus proche de cet objet. On arrive ainsi à déterminer la position et la forme de l'objet 1 dans le quadrilatère ABCD.

Bien entendu, dans le cas de sources ponctuelles émettant des faisceaux coniques, le processus que l'on vient d'expliquer est exactement le même.

Pour accroître la rapidité de la convergence des écarts entre les espaces extérieurs et intérieurs à l'objet, il y a avantage à tenir compte des points connus de l'objet. Ces points connus correspondent par exemple aux points de tangence à l'objet supposé au point T sur la figure 10, de deux rayons issus de deux sources différentes. Dans le cas de trois projections, ou plus, trois rayons au moins passent par un tel point T, dont deux sont tangents à l'objet.

En reportant à partir de ce point T la distance I₁ parcourue dans l'objet 1 par le rayon passant par ce point T, on détermine un second point connu T₁ dans la limite de la précision des mesures. Ce second point connu T₁ permet de déterminer par report de la distance d₁ un troisième point T₃, puis un quatrième T₄ par report de l₂ puis un cinquième par report de d₃.

Grâce à ces points connus de l'objet, on augmente la précision des espaces entre lesquels et situé l'objet 1.

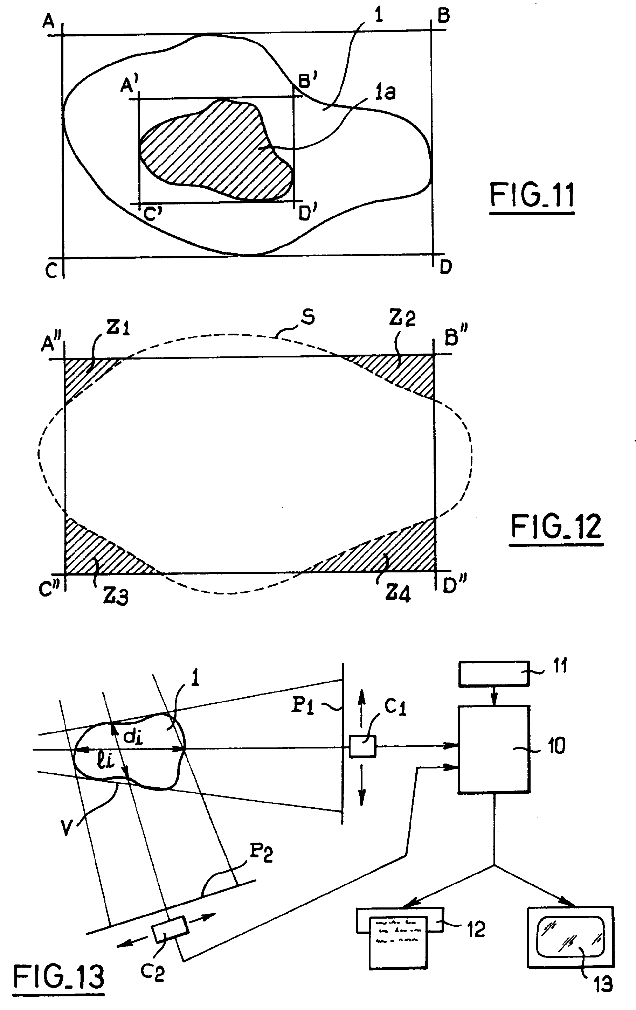

Selon l'invention, où on utilise au moins trois projections, comme illustré par la figure 1, la section du volume V contenant l'objet 1 apparaît sous la forme d'un polygone A˝B˝C˝D˝ (voir figure 12) (correspondant aux deux premières projections) auquel se superpose la section de la surface réglée de contour apparent S de la troisième projection. Cette superposition fait apparaître comme indiqué sur la figure 12 des zones d'exclusion Z₁, Z₂, Z₃, Z₄ extérieures à l'objet, qui permettent ainsi de commencer le procédé sur la base d'un volume contenant l'objet, plus précis que le quadrilatère A˝B˝C˝D˝.

Le procédé que l'on vient de décrire peut également s'appliquer à la reconstitution de la forme et de la position de plusieurs objets adjacents ou contenus les uns dans les autres.

Ainsi, la figure 11 représente un objet plus petit 1a contenu dans un objet plus grand.

Dans ce cas, on commence par déterminer le volume (le rectangle A′B′C′D′ dans l'exemple représenté) qui contient l'objet 1a et les rayons issus des sources qui traversent cet objet 1a.

Ce volume ou rectangle A′B′C′D′ peut être déterminé par la discontinuité dans la transmission des rayons lorsque ceux-ci atteignent le contour apparent de l'objet 1a.

Une fois que le volume contenant l'objet 1a est déterminé, on soustrait celui-ci de l'ensemble de l'objet 1 pour déterminer la forme et la position de celui-ci, en appliquant le procédé décrit ci-dessus. Cette soustraction peut s'effectuer en affectant au volume de l'objet 1a les mêmes caractéristiques que celles de l'objet 1. La forme et la position de l'objet 1 étant connues avec précision, on peut déterminer avec précision la forme et la position de l'objet 1a en appliquant la méthode décrite ci-dessus.

Dans la pratique, les reports des distances dᵢ, dⱼ, dₖ ; lᵢ, lⱼ lₖ pour déterminer des espaces intérieur et extérieur à l'objet de plus en plus proches de celui-ci peuvent être réalisés au moyen d'un ordinateur capable d'effecteur un grand nombre d'opérations en un temps réduit.

La précision et la détermination des positions et formes des objets dépend essentiellement du nombre et la position des différents rayons, de la précision avec laquelle ils sont localisés ainsi que celle de la détermination des distances parcourues dans l'objet. Cette précision dans la localisation et les distances peut être grandement facilitée par l'adjonction de marqueurs de disposition et de position connues dans l'espace à analyser.

Ainsi, un ensemble de quatre marqueurs définissant une sphère de référence, peut être particulièrement approprié. La figure 13 illustre schématiquement le dispositif pour la mise en oeuvre du procédé selon l'invention.

Ce dispositif comprend des capteurs C₁, C₂ capables de mesurer l'intensité des rayons ayant traversé l'objet 1 en chaque point des projections P₁ et P₂.

L'ordinateur 10 relié à ces capteurs C₁, C₂ est capable de convertir ces intensités en distances parcourues à l'intérieur de l'objet 1.

L'ordinateur 10 est également programmé pour déterminer la position de chaque rayon et le volume initial V qui contient l'objet 1 et tous les rayons qui le traversent. Cet ordinateur 10 comprend un moyen d'entrée 11 qui permet d'introduire dans la mémoire de cet ordinateur, les données initiales du dispositif.

Cet ordinateur 10 est relié d'autre part à une imprimante 12 sur laquelle sont imprimés les résultats des calculs et à un écran de visualisation 13 sur lequel on peut faire apparaître la forme définitive de l'objet ainsi que ses formes approchées à un stade déterminé des calculs.

Bien entendu, l'ordinateur 10 pourra, à la demande, reconstituer sur l'imprimante 12 ou l'écran 13, n'importe quelle section de l'objet.

Un logiciel de simulation du procédé de reconstitution selon l'invention a été développé afin d'étudier les performances comparées de différents algorithmes utilisables pour la mise en oeuvre de ce procédé.

Le but ce de logiciel est de reconstituer la forme d'un objet tridimensionnel à partir de vues radiographiques fictives numérisées obtenues à partir d'un modèle initial tridimensionnel de l'objet. Dans ce logiciel, l'étape de prise de vue radiographique effective de l'objet est remplacée par une simulation d'un générateur de rayons X, à titre d'exemple.

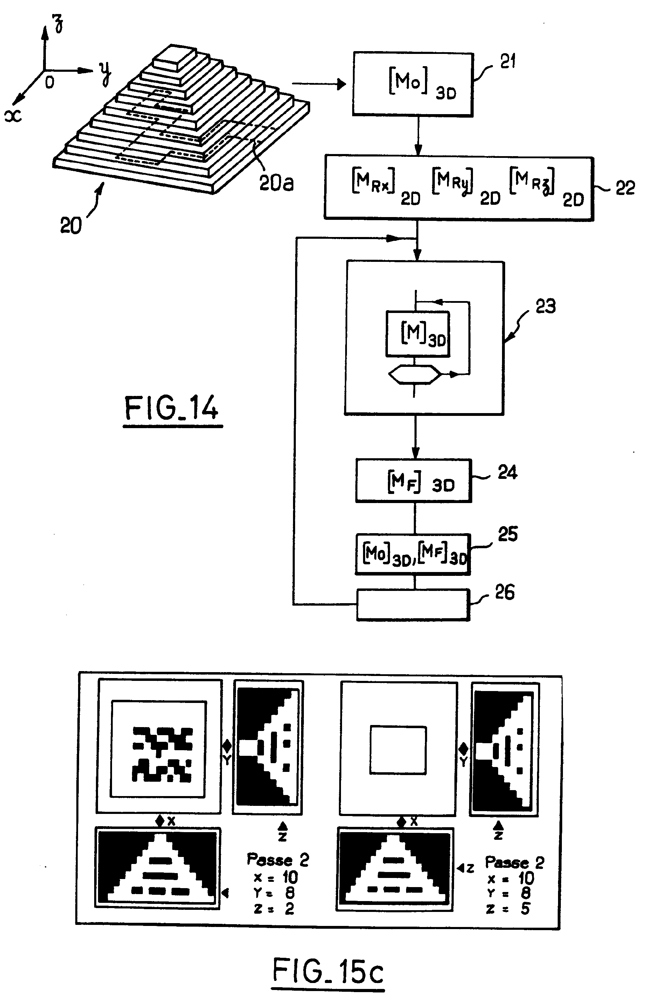

L'objet dont la reconstitution doit être simulée, par exemple une pyramide creuse 20 comprenant un labyrinthe 20a en référence à la figure 14, est modélisé sous la forme d'une matrice de référence tridimensionnelle [Mₒ]_{3D}, au cours d'une étape de modélisation 21. On génère ensuite à partir de cette nature de référence, trois matrices bidimensionnelles [M_{Rx}]_{2D}, [M_{Ry}]_{2D} et [M_{Rz}]_{3D} respectivement équivalentes à trois vues radiographiques de l'objet qui auraient été prises suivant les axes Ox, Oy et Oz (étape 22). Ces trois matrices constituent alors les données de base nécessaires pour l'étape de résolution 23 correspondant au procédé selon l'invention. Cette étape de résolution met en oeuvre un algorithme opérant sur une matrice de calcul [M]_{3D} et itéré un nombre variable de fois jusqu'à ce que soit obtenue une matrice de reconstitution [M_{F}]_{3D} de l'objet concerné (étape 24). La matrice de reconstitution (M_{F}]_{3D} est ensuite comparée (étape 25) à la matrice de référence [M_{O}]_{3D} qui est à l'origine des trois vues radiographiques simulées. A partir du résultat de cette comparaison, les performances de l'algorithme utilisé peuvent être déterminées. Ainsi, plusieurs algorithmes mettant en oeuvre des méthodes déductives ou probabilistiques ont pu être testés avec succès.

Le logiciel de simulation développé à cette fin est doté de nombreuses fonctionnalités permettant de visualiser la reconstitution de l'objet au cours des différentes étapes du procédé, de modifier les conditions initiales, d'effectuer de nombreuses opérations sur les matrices utilisées dans le logiciel et d'identifier après comparaison les éventuelles erreurs de reconstitution.

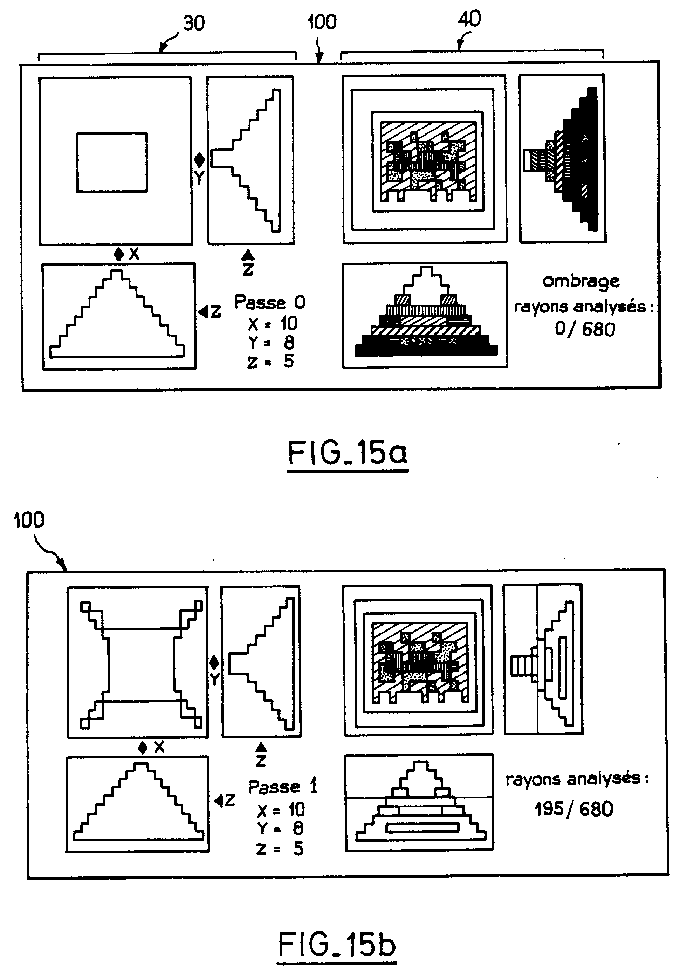

Ecrit pour être utilisé sous forme interactive sur un microordinateur, ce logiciel de simulation du procédé selon l'invention fournit à l'utilisateur des images-écrans, telles que celles représentées de façon très simplifiée à la figure 15. La partie gauche 30 de l'écran 100 représente trois coupes, suivant des valeurs déterminées de x, y et z, de l'image en cours de reconstitution de l'objet étudié, dans le cas présent une pyramide creuse comportant un labyrinthe. Il est possible, grâce à des commandes appropriées, de se déplacer au sein de l'image.

La partie droite 40 de l'écran 100 comprend les trois vues radiographiques suivant les trois axes Ox, Oy et Oz obtenues par simulation d'un générateur de rayons X sur la matrice tridimensionnelle de référence [M_{O}]_{3D}. A chaque cellule élémentaire d'une vue radiographique est associée une densité de gris correspondant à la densité équivalente de pénétration d'un rayon à travers l'objet à l'emplacement de la cellule concernée.

La figure 15a représente une image-écran obtenue lors d'une phase initiale d'exécution du logiciel de simulation, dite d'ombrage, au cours de laquelle les cellules élémentaires correspondant à des densités de matières nulles sont détectées et localisées, permettant de définir les contours extérieurs de l'objet. La figure 15b correspond à une visualisation temporaire de l'image reconstituée à un instant donné au cours d'une première passe de résolution, lorsque seulement 195 rayons sur 680 ont été analysés. A l'issue d'une deuxième passe et après analyse de l'ensemble des rayons traversant les différentes cellules de l'objet, on obtient une image reconstituée finale de la pyramide creuse, en référence à la figure 15c, qui représente, à titre d'exemple, les trois coupes de l'image reconstituée suivant (x, y, z) = (10, 8, 5) et (x, y, z) = (10, 8, 2). A ce stade de l'exécution du logiciel, il est tout à fait possible d'explorer l'ensemble de l'image tridimensionnelle ainsi obtenue et de la comparer avec l'image tridimensionnelle de référence.

Il faut noter que les images-écrans générées par ce logiciel de simulation font apparaître d'autres fenêtres, et en particulier, des fenêtres de commande, non représentées sur les figures 15a à 15c.

## Revendications

1. Procédé mis en oeuvre au moyen d'un ordinateur permettant de reconstituer la forme et la position d'objets (1, 1a) dans l'espace à partir d'au moins trois projections (P₁, P₂, P₃) de ces objets obtenues par transmission d'ondes influencées par le passage dans ces objets à partir de sources (S₁, S₂, S₃) de ces ondes, le procédé comportant les étapes suivantes :
A/ on détermine pour chaque point de ces trois projections (P₁, P₂, P₃) la distance (dᵢ, dⱼ, dₖ ; lᵢ, lⱼ, lₖ) parcourue à travers un objet (1) par chaque rayon issu d'une des sources,
B/ on détermine le volume (V ; V_{A} V_{B} V_{C} V_{D}) qui contient l'objet (1) et les rayons issus des trois sources (S₁, S₂, S₃) qui traversent cet objet,
C/ on reporte les distances (dᵢ, dⱼ, dₖ ; lᵢ, lⱼ, lₖ) parcourues par les rayons dans l'objet (1) à partir des limites extérieures du volume (V) contenant l'objet,
D/ on détermine les espaces pour lesquels il y a recouvrement des distances portées sur les rayons à partir des limites du volume précité pour définir un premier espace minimum (E₁, E₂) qui est nécessairement à l'intérieur de l'objet,
E/ on reporte les distances parcourues par les rayons nécessairement dans l'objet à partir des limites extérieures de ce premier espace (E₁, E₂) précité pour définir un second espace (E₃) qui est nécessairement à l'extérieur de l'objet et qui est plus restreint que le volume extérieur (V) précité.
F/ on reporte les distances parcourues par les rayons dans l'objet à partir des limites extérieures du second espace (E₃) ci-dessus pour définir un troisième espace (E₄) qui est nécessairement à l'intérieur de l'objet et qui est plus étendu que le premier espace,
G/ à partir de ce troisième espace (E₄), on procède, comme dans l'étape E pour définir un quatrième espace (E₅) qui est nécessairement à l'extérieur de l'objet et qui est plus restreint que le second espace,
H/ on reprend les étapes E et F de façon à déterminer par récurrence des espaces extérieurs et intérieurs à l'objet de plus en plus proches de cet objet et on détermine ainsi la forme et la position de celui-ci, et si des points connus (T) du contour de l'objet existent, on détermine à partir des points connus (T) du contour de l'objet, en particulier ceux correspondant aux points de tangence à l'objet de deux rayons issus de deux sources différentes, d'autres points (T₁, T₂, T₃) du contour de ce dernier, en reportant à partir des points connus la distance (d₁, l₁, d₂, l₂) parcourue dans cet objet par les rayons passant par ces points connus, les points (T₁, T₂, T₃) ainsi déterminés étant utilisés pour augmenter la précision des espaces entre lesquels est situé l'objet.

2. Procédé conforme à la revendications 1 appliqué à la reconstitution de la forme et de la position de plusieurs objets (1, 1a), séparés ou non, dans lequel on détermine le volume (A′B′C′D′) qui contient chacun des objets et les rayons issus des sources qui traversent cet objet, et on détermine à partir de ce volume la forme et la position de cet objet comme indiqué dans les revendications précédentes.

3. Procédé conforme à la revendication 2, dans lequel on détermine le volume de chaque objet par la discontinuité dans la transmission des rayons lorsque ceux-ci atteignent le contour apparent de l'objet.

4. Procédé conforme à la revendication 3, dans lequel le volume (A′B′C′D′) contenant un objet (1a) particulier est soustrait de l'ensemble pour déterminer successivement la forme et la position des autres objets.

5. Ordinateur permettant de reconstituer la forme et la position d'objets (1, 1a) dans l'espace comprenant des moyens pour obtenir au moins trois projections (P₁, P₂, P₃) de ces objets par transmission d'ondes influencées par le passage dans ces objets à partir de sources (S₁, S₂, S₃) de ces ondes, le dispositif comportant :
A/ des moyens pour déterminer pour chaque point de ces projections (P₁, P₂, P₃), la distance (dᵢ, lᵢ) parcourue à travers les objets par chaque rayon issu d'une des sources,
B/ des moyens pour déterminer le volume (V) qui contient l'objet et les rayons issus des trois sources (S₁, S₂, S₃) qui traversent cet objet,
C/ des moyens pour reporter les distances (dᵢ, lᵢ) parcourues par les rayons dans l'objet à partir des limites extérieures du volume (V) contenant l'objet,
D/ des moyens pour déterminer les recouvrements des distances portées sur les rayons à partir du volume précité pour définir un premier espace minimum (E₁, E₂) qui est nécessairement à l'intérieur de l'objet,
E/ des moyens pour reporter les distances parcourues par les rayons dans l'objet à partir des limites extérieures du premier espace (E₁, E₂) précité pour définir un second espace (E₃) qui est nécessairement à l'extérieur de l'objet et qui est plus restreint que le volume (V) précité,
F/ des moyens pour reporter les distances parcourues par les rayons dans l'objet à partir des limites extérieures du second espace (E₃) ci-dessus pour définir un troisième espace (E₄) qui est nécessairement à l'intérieur de l'objet et qui est plus étendu que le premier espace,
G/ des moyens pour déterminer de la même manière que les moyens E un quatrième espace (E₅) qui est nécessairement à l'extérieur de l'objet et qui est plus restreint que le second espace (E₃),
H/ des moyens pour déterminer par récurrence des espaces extérieurs et intérieurs à l'objet de plus en plus proches de cet objet,
cet ordinateur comprenant en plus des moyens pour déterminer à partir des points connus (T) du contour de l'objet, en particulier ceux correspondant aux points de tangence à l'objet de deux rayons issus de deux sources différentes, d'autres points (T₁, T₂, T₃) du contour de ce dernier, en reportant à partir des points connus la distance (d₁, l₁, d₂, l₂) parcourue dans cet objet par les rayons passant par ces points connus, les points (T₁, T₂ T₃) ainsi déterminés étant utilisés pour augmenter la précision des espaces entre lesquels est situé l'objet.

6. Ordinateur conforme à la revendication 5, dans lequel les moyens selon B/ à H/ sont constitués par un ordinateur (10) ayant en mémoire, pour chaque point des projections (P₁, P₂), la distance (dᵢ, lᵢ) parcourue à travers un objet par chaque rayon issu d'une des sources, la position et la forme du volume (V) qui contient l'objet et les rayons issus des deux sources qui traversent cet objet et qui est programmé pour calculer les points d'intersection entre les rayons et ce volume (V), pour reporter les distances (dᵢ, lᵢ) précitées à partir de ces points d'intersection, calculer les positions obtenues après ce report puis reporter successivement les distances à partir de ces nouvelles positions jusqu'à ce que les écarts entre les nouvelles positions calculées soient les plus proches possibles des positions calculées dans le cycle précédent.

7. Ordinateur conforme à la revendication 6, dans lequel l'ordinateur (10) est programmé pour déterminer la forme et la position d'une section de l'objet par n'importe quel plan de celui-ci.

8. Ordinateur conforme à la revendication 7, dans lequel l'ordinateur (10) est associé à des moyens d'affichage (12) et/ou de visualisation (13) du résultat de ses calculs.

9. Ordinateur conforme à l'une des revendications 5 à 8, comprenant des marqueurs dont la disposition et la position sont connues dans le volume à analyser.

## Patentansprüche

1. Durch eine Datenverarbeitungsanlage ausgeführtes Verfahren, mit dem die Form und die Position von Objekten (1, 1a) im Raum ausgehend von wenigstens drei Projektionen (P₁, P₂, P₃) dieser Objekte wiederhergestellt werden können, die durch die Übertragung von Wellen erhalten werden, die durch das Durchlaufen dieser Objekte ausgehend von Quellen (S₁, S₂, S₃) dieser Wellen beeinflußt werden, wobei das Verfahren die folgenden Schritte enthält:
A/ Man bestimmt für jeden Punkt dieser drei Projektionen (P₁, P₂, P₃) die Strecke (dᵢ, dⱼ, dₖ; lᵢ, lⱼ, lₖ), die von jedem von einer der Quellen abgegebenen Strahl durch ein Objekt (1) zurückgelegt wurde,
B/ man bestimmt das Volumen (V; V_{A} V_{B} V_{C} V_{D}), das das Objekt (1) und die von den drei Quellen (S₁, S₂, S₃) gelieferten, durch dieses Objekt laufenden Strahlen enthält,
C/ man zeichnet die von den Strahlen in dem Objekt (1) ausgehend von den äußeren Grenzen des das Objekt enthaltenden Volumens (V) zurückgelegten Strecken (dᵢ, dⱼ, dₖ; lᵢ, lⱼ, lₖ) auf,
D/ man bestimmt die Räume, für die es eine Überdeckung der auf den Strahlen ausgehend von den Grenzen des zuvor genannten Volumens aufgetragenen Strecken gibt, um einen ersten kleinsten Raum (E₁, E₂) zu definieren, der im Inneren des Objekts erforderlich ist,
E/ man zeichnet die von den Strahlen notwendigerweise in dem Objekt ausgehend von den äußeren Grenzen dieses zuvor genannten ersten Raums (E₁, E₂) zurückgelegten Strecken auf, um einen zweiten Raum (E₃) zu definieren, der notgedrungen außerhalb des Objekts liegt und der stärker als das zuvor genannte äußere Volumen (V) begrenzt ist,
F/ man zeichnet die von den Strahlen in dem Objekt ausgehend von den äuBeren Grenzen des zweiten obigen Raums (E₃) zurückgelegten Strecken auf, um einen dritten Raum (E₄) zu definieren, der notgedrungen im Inneren des Objekts liegt und der ausgedehnter als der erste Raum ist,
G/ ausgehend von diesem dritten Raum (E₄) verfährt man wie bei dem Schritt E, um einen vierten Raum (E₅) zu definieren, der notgedrungen außerhalb des Objekts liegt und der stärker begrenzt ist als der zweite Raum,
H/ man führt wieder die Schritte E und F aus, um durch Rekursion die Räume außerhalb und innerhalb des Objekts in zunehmender Nähe zu diesem Objekt zu bestimmen, und man bestimmt so dessen Form und dessen Position, und, wenn bekannte Punkte (T) der Kontur des Objekts vorliegen, bestimmt man ausgehend von den bekannten Punkten (T) der Kontur des Objekts insbesondere jene von weiteren Punkten (T₁, T₂, T₃) der Kontur dieses letzteren, die den Tangentenpunkten zweier von zwei unterschiedlichen Quellen gelieferter Strahlen an dem Objekt entsprechen, indem man ausgehend von den bekannten Punkten die Strecke (d₁, l₁, d₂, l₂) aufzeichnet, die von den an diesen bekannten Punkten vorbeilaufenden Strahlen in diesem Objekt zurückgelegt wurde, wobei die so bestimmten Punkte (T₁, T₂, T₃) dazu verwendet werden, die Genauigkeit der Räume, zwischen denen das Objekt liegt, zu erhöhen.

2. Verfahren nach Anspruch 1, das zur Wiederherstellung der Form und der Position mehrerer Objekte (1, 1a), getrennt, oder nicht, angewandt wird und bei dem man das Volumen (A′B′C′D′) bestimmt, das jedes der Objekte und die von den Quellen gelieferten Strahlen enthält, die durch dieses Objekt verlaufen, und bei dem man ausgehend von diesem Volumen die Form und die Position dieses Objekts wie in den vorhergehenden Ansprüchen angegeben bestimmt.

3. Verfahren nach Anspruch 2, bei dem man das Volumen jedes Objekts durch die Diskontinuität bei der Übertragung der Strahlen bestimmt, wenn diese die auftretende Kontur des Objekts erreichen.

4. Verfahren nach Anspruch 3, bei dem das ein einzelnes Objekt (1a) enthaltende Volumen (A′B′C′D′) von dem Ganzen subtrahiert wird, um aufeinanderfolgend die Form und die Position der anderen Objekte zu bestimmen.

5. Datenverarbeitungsanlage, durch die die Form und die Position von Objekten (1, 1a) in dem Raum wiederhergestellt werden können, mit Mittel zur Erlangung von wenigstens drei Projektionen (P₁, P₂, P₃) dieser Objekte durch eine Übertragung von Wellen, die durch das Durchlaufen dieser Objekte ausgehend von Quellen (S₁, S₂, S₃) dieser Wellen beeinflußt werden, wobei die Vorrichtung folgendes enthält:
A/ Mittel, um für jeden Punkt dieser Projektionen (P₁, P₂, P₃) die Strecke (dᵢ, lᵢ) zu bestimmen, die von jedem von einer der Quellen gelieferten Strahl durch diese Objekte hindurch zurückgelegt wurde,
B/ Mittel zur Bestimmung des Volumens (V), das das Objekt und die von den drei Quellen (S₁, S₂, S₃) gelieferten, dieses Objekt durchquerenden Strahlen enthält,
C/ Mittel, um die Strecken (dᵢ, lᵢ) aufzuzeichnen, die von den Strahlen in dem Objekt ausgehend von den äußeren Grenzen des Volumens (V) zurückgelegt wurden, das das Objekt enthält,
D/ Mittel zur Bestimmung der Überdeckungen der auf den Strahlen ausgehend von dem zuvor genannten Volumen aufgetragenen Strecken, um einen ersten kleinsten Raum (E₁, E₂) zu definieren, der notgedrungen im Inneren des Objekts liegt,
E/ Mittel zum Aufzeichnen der von den Strahlen in dem Objekt ausgehend von den äußeren Grenzen des zuvor genannten ersten Raums (E₁, E₂) zurückgelegten Strecken, um einen zweiten Raum (E₃) zu definieren, der notgedrungen außerhalb des Objekts liegt und der stärker begrenzt ist als das zuvor genannte Volumen (V),
F/ Mittel zum Aufzeichnen der von den Strahlen in dem Objekt ausgehend von den äußeren Grenzen des zweiten obigen Raums (E₃) zurückgelegten Strecken, um einen dritten Raum (E₄) zu definieren, der notgedrungen im Inneren des Objekts liegt und der ausgedehnter ist als der erste Raum,
G/ Mittel, um auf die gleiche Weise wie die durch die Mittel E einen vierten Raum (E₅) zu bestimmen, der notgedrungen außerhalb des Objekts liegt und der stärker begrenzt ist als der zweite Raum (E₃),
H/ Mittel, um durch Rekursion Räume außerhalb und innerhalb des Objekts in zunehmender Nähe zu diesem Objekt zu bestimmen, wobei diese Datenverarbeitungsanlage überdies Mittel enthält, um ausgehend von den bekannten Punkten (T) der Kontur des Objekts insbesondere jene von weiteren Punkten (T₁, T₂, T₃) zu bestimmen, die den Tangentenpunkten zweier von zwei unterschiedlichen Quellen gelieferter Strahlen an dem Objekt entsprechen, indem ausgehend von den bekannten Punkten die Strecke (d₁, l₁, d₂, l₂) aufgezeichnet wird, die von den an diesen bekannten Punkten vorbeilaufenden Strahlen in diesem Objekt zurückgelegt wurde, wobei die so bestimmten Punkte (T₁, T₂, T₃) dazu verwendet werden, die Genauigkeit der Räume, zwischen denen das Objekt liegt, zu erhöhen.

6. Datenverarbeitungsanlage nach Anspruch 5, bei der die Mittel gemäß B/ bis H/ durch eine Datenverarbeitungsanlage (10) gebildet sind, die für jeden Punkt der Projektionen (P₁, P₂) die von jedem von einer der Quellen gelieferten Strahl durch das Objekt hindurch zurückgelegte Strecke (dᵢ, lᵢ), die Position und die Form des Volumens (V) gespeichert hält, das das Objekt und die von den beiden Quellen gelieferten, dieses Objekt durchdringenden Strahlen enthält, und die dafür programmiert ist, die Schnittpunkte zwischen den Strahlen und diesem Volumen (V) zu berechnen, die zuvor genannten Strecken (dᵢ, lᵢ) ausgehend von diesen Schnittpunkte aufzuzeichnen, die erhaltenen Positionen nach dieser Aufzeichnung zu berechnen und dann aufeinanderfolgend die Strecken ausgehend von diesen neuen Positionen aufzuzeichnen, bis die Abstände zwischen den neuen, berechneten Positionen von den in dem vorhergehenden Zyklus berechneten Positionen die am nächsten gelegenen sind.

7. Datenverarbeitungsanlage nach Anspruch 6, bei der die Datenverarbeitungsanlage (10) dafür programmiert ist, die Form und die Position eines Schnitts des Objekts durch irgendeine Ebene davon zu bestimmen.

8. Datenverarbeitungsanlage nach Anspruch 7, bei der der Datenverarbeitungsanlage (10) Anzeigemittel (12) und/oder Sichtbarmachungsmittel (13) zur Wiedergabe des Ergebnisses dieser Berechnungen zugeordnet sind.

9. Datenverarbeitungsanlage nach einem der Ansprüche 5 bis 8, mit Markierungen, deren Verteilung und Position in dem zu analysierenden Volumen bekannt sind.

## Claims

1. A method, performed by means of a computer, of reconstructing the shape and position of objects (1, 1a) in space from at least three projections (P₁, P₂, P₃) of the objects obtained by transmission of waves starting from sources (S₁, S₂, S₃) of said waves and influenced by travel in the objects, the process comprising the following steps:
A) at each point of the three projections (P₁, P₂, P₃) the distance (dᵢ, dⱼ, dₖ; lₗ, lⱼ, lₖ) travelled through the object (1) by each ray from one of the sources is determined,
B) the volume (V; Vₐ, V_{B}, V_{C}, V_{D}) containing the object (1) and the rays from the three sources (S₁, S₂, S₃) and travelling through the object is determined,
C) the distances (dᵢ, dⱼ, dₖ; lᵢ, lⱼ, lₖ) travelled by the rays in the object (1) starting from the outer limits of the volume (V) containing the object, are plotted,
D) the spaces in which there is overlapping of the plotted distances on the rays starting from the limits of the said volume are determined so as to define a first minimum space (E₁, E₂), which is necessarily inside the object,
E) the distances travelled by the rays necessarily in the object, starting from the outer limits of the said first space (E₁, E₂), are plotted so as to define a second space (E₃) which is necessarily outside the object and is more restricted than the said outer volume (V),
F) the distances travelled by the rays in the object starting from the outer limits of the said second space (E₃) are plotted so as to define a third space (E₄), which is necessarily inside the object and is more extensive than the first space,
G) starting from said third space (E₄), the procedure in step E is followed so as to define a fourth space (E₅) which is necessarily outside the object and is more restricted than the second space, and
H) steps E and F are repeated so as by recursion to determine spaces outside and inside the object and increasingly close to the object, so as to determine the shape and position thereof, and if known points (T) of the contour of the object exist, the known points (T) of the contour of the object, more particularly those corresponding to points where the object is touched by two rays from two different sources, are used to determine other points (T₁, T₂, T₃) of the contour of the object, by using the known points to plot the distance (d₁, l₁, d₂, l₂) travelled in the object by the rays passing through said known points, the points (T₁, T₂, T₃) thus determined being used to increase the accuracy of the spaces within which the object is situated.

2. A method according to claim 1, applied to reconstruction of the shape and position of a number of objects (1, 1a) which may or may not be separated, and wherein the volume (A′, B′, C′, D′) containing each object and the rays from the sources travelling through said object are determined, and said volume is used to determine the shape and position of said object as indicated in the preceding claims.

3. A method according to claim 2, wherein the volume of each object is determined by the discontinuity in the transmission of the rays when they reach the apparent contour of the object.

4. A method according to claim 3 wherein the volume (A′, B′, C′, D′) containing a particular object (1a) is subtracted from the total in order successively to determine the shape and position of the other objects.

5. A computer for reconstructing the shape and position of objects (1, 1a) in space, comprising means for obtaining at least three projections (P₁, P₂, P₃) of said objects by transmission of waves influenced by passing through said objects from sources (S₁, S₂, S₃) of said waves, the apparatus comprising
A) means for determining, for each point of said projections (P₁, P₂, P₃), the distance (d₁, l₁) travelled through the objects by each ray from one of the sources,
B) means for determining the volume (V) which contains the object and the rays from the three sources (S₁, S₂, S₃) and travelling through the object,
C) means for plotting the distances (d₁, l₁) travelled by the rays in the object, starting from the outer limits of the volume (V) containing the object,
D) means for determining the overlaps of the plotted distances on the rays, starting from the said volume, in order to define a first minimum space (E₁, E₂) which is necessarily inside the object,
E) means for plotting the distances travelled by the rays in the object, starting from the outer limits of the said first space (E₁, E₂), in order to define a second space (E₃) which is necessarily outside the object and is more restricted than the said volume (V),
F) means for plotting the distances travelled by the rays in the object, starting from the outer limits of the said second space (E₃), in order to define a third space (E₄) which is necessarily inside the object and is more extensive than the first space,
G) means for determining, in the same manner as means (E), a fourth space (E₅) which is necessarily outside the object and is more restricted than the second space (E₃),
H) recursion means for determining spaces outside and inside the object and increasingly close to the object,
I) said computer also comprising means for determining from known points (T) of the contour of the object, more particularly those corresponding to points where the object is touched by two rays from two different sources, other points (T₁, T₂, T₃) of the contour of the object, by plotting from the known points the distance (d₁, l₁, d₂, l₂) travelled in the object by rays passing through said known points, the points (T₁, T₂, T₃) thus determined being used to increase the accuracy of the spaces between which the object is situated.

6. A computer according to claim 5, wherein the means according to B) to H) comprise a computer (10) which, for each point of the projections (P₁, P₂), stores the distance (dᵢ, lᵢ) travelled through an object by each ray from one of the sources, the position and the shape of the volume (V) containing the object and the rays from the two sources and travelling through the object, the computer being programmed to calculate the points of intersection between the rays and said volume (V), to plot the said distances (dᵢ, lᵢ) from said points of intersection, to calculate the positions obtained after plotting and then successively to plot the distances from said new positions until the deviations between the calculated new positions are as close as possible to the positions calculated in the preceding cycle.

7. A computer according to claim 6, wherein the computer (10) is programmed to determine the shape and position of a section of the object through any plane thereof.

8. A computer according to claim 7, wherein the computer (10) is associated with means for displaying (12) and/or visualising (13) the result of the calculations.

9. A computer according to any of claims 5 to 8, comprising markers having a known position and arrangement in the volume for analysis.
